# EUROPEAN PATENT APPLICATION

(11) **EP 2 075 344 A1**
(43) Date of publication of application: **01.07.2009**
(21) Application number: 07450241.0
(22) Date of filing: 21.12.2007
(51) Int. Cl.: C12Q 1/68

(54) **A method of a diagnostic assay**

(71) Applicant: Mayer, Gert, 6020 Innsbruck (AT)
(72) Inventor: Mayer, Gert, 6020 Innsbruck (AT); Koppelstätter, Christian, 6020 Innsbruck (AT); Perco, Paul, 1080 Vienna (AT); Schratzberger, Gabriele, 6020 Innsbruck (AT)
(74) Representative: Redl, Gerda

(57) **Abstract**

The present invention provides a method of a diagnostic or prognostic assay for determining organ quality, in particular for suitability of organ transplantation, in a donor organ sample, which comprises determining an amount of CDKN2A and the chronological age or telomere length in the sample.

## Description

The present invention relates to the field of organ transplantation and organ quality determination.

The incidence of end stage renal disease is rising continuously and dramatically. In 1994 4345 patients underwent renal replacement therapy in Austria. Only 10 years later the prevalence increased to 6564. As the prognosis of patients on dialysis is still poor, transplantation is attempted whenever possible, reducing long term mortality by 48 - 82%. Within the Eurotransplant organisation approximately 12000 patients are currently registered on the renal waiting list, but only about 3000 grafts can be allocated each year because of donor shortage. In an effort to increase organ supply kidneys from so called "expanded criteria" donors are transplanted more frequently, although the outcome on average is worse. There is no uniform consensus on which specific donor factors preclude a long term success but chronological donor age definitely is among the most important parameters affecting late allograft function and even recipient mortality. This fact is particularly remarkable as.the expansion of the donor pool during the last decades has been mostly achieved by extending the upper age limit. From 1977 to 1982 a donor older than 30 years was the most likely reason for transplant physicians to reject an offer, in 1996 this threshold shifted to 60 years. Within Eurotransplant 11.1% of the donors were older than 65 years in 1998, but 16.5% in 2003.

Despite the overall negative impact of donor age the Eurotransplant Senior Program, which exclusively allocates kidneys from donors older than 65 years to recipients older than 65 years has reported an acceptable outcome after one year of follow up. Thus a significant number of organs from elderly (as judged by chronological age) donors maintain excellent function for a prolonged period of time.

As chronological age does not provide all information necessary with respect to prognosis of organ function, and an estimate of "biological" organ age could be preferable. Over the last decades research efforts resulted in the availability of several biological age markers such as telomere length. Telomeres are the non-coding regions at the end of the chromosomes. Usually, about 10 to 13 kilo base pairs long in newborn humans, they are shortened by each cell cycle and potentially also by environmental stress factors. Once a critical telomere length is reached cells enter a state of stable growth/replication arrest called senescence.

Yet, telomere length is a marker that correlates with chronological age and provides a similar prognosis of organ function after transplantation. Therefore, it is a goal of the present invention to provide the means to determine further markers of biological age and organ function to allow an even broader access to donor organs with positive outcome.

The present invention provides a method of a diagnostic or prognostic assay for determining organ quality, in particular for suitability of organ transplantation, in a donor organ sample, in particular prior to engraftment, which comprises determining an amount of CDKN2A and the chronological age or telomere length in the sample. Thus, the invention provides the combined use of one of the most suitable surrogate markers of biological organ transplant age, i.e. telomere length and chronological age in combination with CDKN2A. None of the markers alone has the predictive quality to allow a solid estimate of expected transplant function. The chronological age can of course be determined from the donating person's age and does not necessitate another measurement.

Both CDKN1A and CDKN2A are cell cycle inhibitors that have been shown to induce a senescent phenotype. While CDKN1A activity is related to telomere shortening via the p53 pathway, CDKN2A is linked to a telomere-shortening independent form of stress induced senescence. For the present invention age, telomere length and the gene expression levels of CDKN2A and CDKN1A in renal biopsies obtained immediately before engraftment were determined and their power to predict serum creatinine levels as an indicator of renal excretory function one year after transplantation was compared. Predictive models were derived from this data, which were then tested in a prospective study. Surprisingly, it has been found that although the use of CDKN1A as a marker did not increase prognostic power as provided by chronological donor age or telomere length, addition of information derived on CDKN2A significantly improved the predictive models derived from chronological age or telomere length.

Even though advanced chronological donor age is on average the most important single negative predictor of long term outcome after renal transplantation, many organs from elderly donors maintain excellent function for years. As the rising demand for donor kidneys can only be met by accepting organs from elderly subjects more frequently, transplant physicians and their patients are looking for a better way to assess age related "organ quality", ideally prior to engraftment. Markers of cellular senescence and thus surrogates for "biological age obtained in zero hour biopsies were investigated to provide additional or better information on what can be expected in terms of late postoperative allograft function when compared to other, conventionally used parameters, including chronological donor age.

Historically, telomere shortening has been considered a hallmark of the classical form of replicative senescence. Once an individual chromosome is not "capped" any more by a telomere exceeding a critical length, or the associated multi-component protein assembly is disturbed, the situation is sensed as a DNA strand break and p53 levels increase, resulting in the transcriptional activation of CDKN1A, a cyclin kinase inhibitor, which mediates a cell cycle arrest in the G1 phase. Recently, the concept of senescence has been expanded to additionally include cell cycle arrest induced by DNA damaging agents, oxidative or "oncogenic" stress, and other metabolic perturbations (so called STASIS, i.e. stimulation and stress induced senescent like arrest). Telomere shortening has been observed under these circumstances as well, but, additionally, the activation of CDKN2A acts as a second dominant factor to limit growth.

The organ investigated for quality can be any organ of a subject. In preferred embodiments the organ is a kidney. However the markers are also expressed and thus could also be applied to other organs or tissues such as bone marrow, bowel, spleen, thymus, brain, spinal cord, muscle, in particular skeletal muscle, heart, liver, pancreas, prostate and lung. Thus, other preferred organs are selected therefrom.

The most prominent marker assessing renal function is serum creatinine and thus serum creatinine was used one year after transplantation as the dependent variable of interest. Allograft function at this point in time is a very strong predictor for graft half life and is influenced by the same risk factors which have been associated with the incidence of chronic allograft nephropathy. A baseline value of 1.2 mg/dl in the serum is considered a healthy reference value, whereas values elevated by 50% are an indicator of organ failure (US 7,141,382). The present invention provides a prediction of creatinine values after an organ transplantation by use of the above combination of determining chronological age/telomere length with CDKN2A. It was found that after one year of transplant, creatinine values (mg/dl) correlate to about 0.014 times the chronological donor age plus about 0.024 CDKN2A concentration (e.g. as measured in relation to housekeeping genes such as GAPDH) in the sample and also correlates to about -0.13 times the telomere length (T/S ratio) plus about 0.04 times CDKN2A concentration (e.g. as measured in relation to housekeeping genes such as GAPDH).

Furthermore the invention also relates to the method of predicting creatinine levels in the blood of a subject which received a kidney transplant, comprising determining an amount of CDKN2A and either the chronological age or telomere length, or both, in the donor kidney sample prior to transplantation. The values of CDKN2A together with chronological age and/or telomere length in order to predict serum creatinine, e.g. after one year of the transplantation, can then be correlated with data of creatinine levels and to establish a regression function. Such a formula is, e.g., given in example 6 below. In general, the present invention also provides a method of predicting transplant organ (e.g. kidney) function, in particular, as determined by creatinine levels in blood of a subject which received a organ transplant, comprising determining an amount of CDKN2A and the chronological age or telomere length in the donor organ sample prior to transplantation, correlating a known organ function with CDKN2A and the chronological age or telomere length, and calculating the predicted organ function from the correlation with the determined amounts of CDKN2A and the chronological age or telomere length. In particular, organ function can be predicted for at least or up to, e.g., one week, one month, four months, six months, eight months, one year, two years or even three or more years after transplantation.

In preferred embodiments the sample is a biopsy sample which can be obtained from the organ prior to transplantation. As most organs are from recently deceased persons, the donor organ as well as its sample can be provided isolated from the donor person. The transplant receiving subject as well as the organ donor can be any animal, in particular mammals, such as a human, mouse, rat, hamster, cat, dog, horse, cow, pig, etc.

According to the present invention the amount of CDKN2A can be determined by any routine method known in the art, including determining the CDKN2A expression, preferably by determining the CDKN2A mRNA concentration. To this extend, mRNA of the sample of the organ can be isolated after sample homogenisation and hybridized with CDKN2A specific probes, in particular on a microarray platform with or without amplification, or primers for PCR-based detection methods, e.g. PCR extension labelling with probes specific for a portion of the mRNA.

In other embodiments the amount of CDKN2A is determined by measurement of the CDKN2A protein concentration, e.g. with CDKN2A specific antibodies or binding partners. The binding event can, e.g., be detected by competitive or non-competitive methods and include the use of labeled CDKN2A specific moieties (e.g. antibodies) or labelled competitive moieties (including a labeled CDKN2A standard) which compete with CDKN2A proteins for the binding event.

In further aspects the present invention relates to a method for prediction of organ transplant failure, comprising determining an amount of CDKN2A and the chronological age or telomere length in the donor organ sample, and comparing the determined amounts to amounts of CDKN2A and the chronological age and/or telomere length with organ function after transplantation of healthy patients, wherein the chance of organ transplant failure is increased if the determined amounts in the donor sample are greater than the amounts of the healthy transplant patients. In preferred embodiments the amounts in the donor sample, the chronological age and/or the telomere length are e.g. by at least 20%, 40%, 60%, 80%, 100%, 150% or 200% greater than the amounts, the chronological age and/or the telomere length of the healthy patients. Also provided is a method for prediction of organ transplant failure, comprising predicting the creatinine level as mentioned above, wherein the chance of organ transplant failure is considered increased if predicted creatinine values are greater than 1.8 mg/dl or 2.0 mg/dl. As mentioned above, organ function after allograft transplantation can be correlated to the inventive marker combination, the CDKN2A amount and either (or both) chronological age and telomere length in the donor organ. This correlation gives a direct and linear or non-linear measure of the chances of success of an organ transplantation. The CDKN2A amount stands in an intricate relationship with the other markers and gives a satisfactory expectation of the organ function, e.g. measured via the serum creatinine level. In specific embodiments the invention can be used to diagnose or prognose chronic or acute organ transplant failure.

Preferably, the telomere length is determined by determining the T/S-value, i.e. the relation between telomere repeat copy number and single copy number as described by Cawthon (Nucleic Acids Res 2002;30:e47).

In another aspect the present invention provides a set comprising means for the determination of CDKN2A mRNA or CDKN2A protein, preferably by CDKN2A mRNA specific PCR primers, CDKN2A hybridizing oligonucleotides or CDKN2A specific antibodies, and means for the determination of telomere length, preferably primers for the determination of the T/S-value.

The present invention is further illustrated by the following figures and examples without being limited thereto.

### Figures:

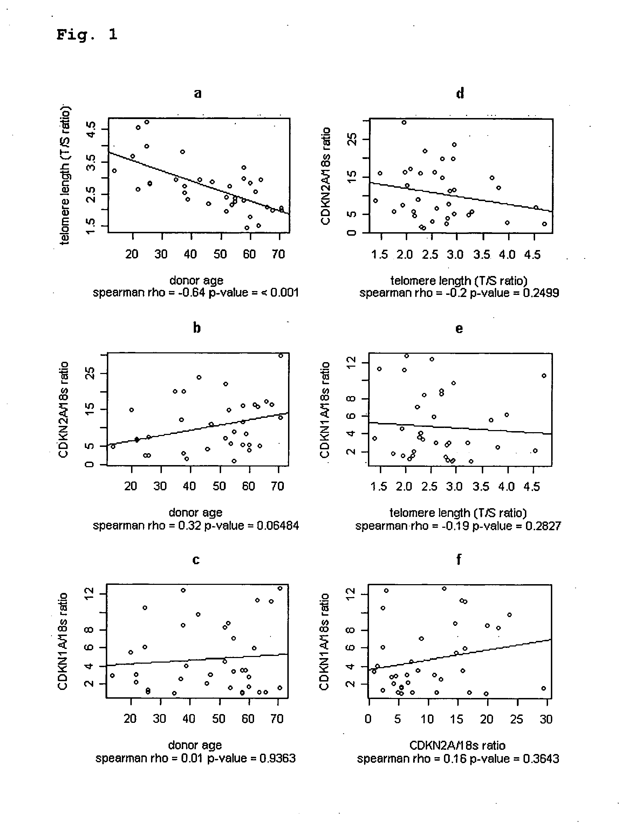

Fig. 1: Correlation between donor age and (a) telomere length, (b) CDKN2A and (c) CDKN1A. The correlation donor age with telomere length was significant. With CDKN2A a trend was noted. Telomere length showed neither a correlation with CDKN2A (d) nor with CDKN1A (e). The expression of CDKN1A was not correlated with the expression of CDKN2A (f).

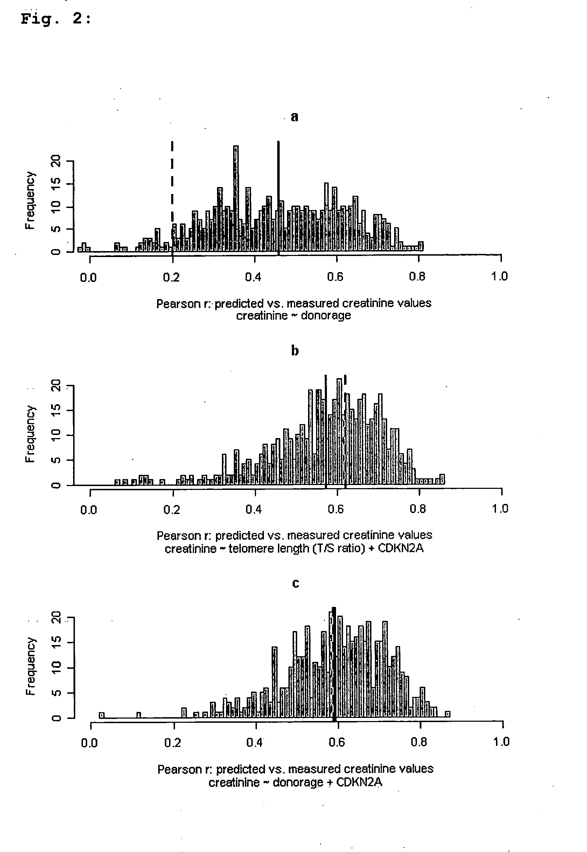

Fig. 2: Predicted versus measured serum creatinine values in the cross-validation analysis 500 runs: Histograms of pearson correlation values between predicted and measured creatinine values after 500 runs in the cross-validation analysis using 35 randomly picked samples for training and the remaining 19 for validation. Figure 2a depicts the distribution of correlations using donor age alone, whereas the results of the models consisting of telomere length and CDKN2A expression as well as donor age and CDKN2A expression for creatinine prediction are given in figures 2b and 2c, respectively. The black solid lines depict the mean correlation values after 500 runs, the black dotted lines the actually observed values for the prospective study data set.

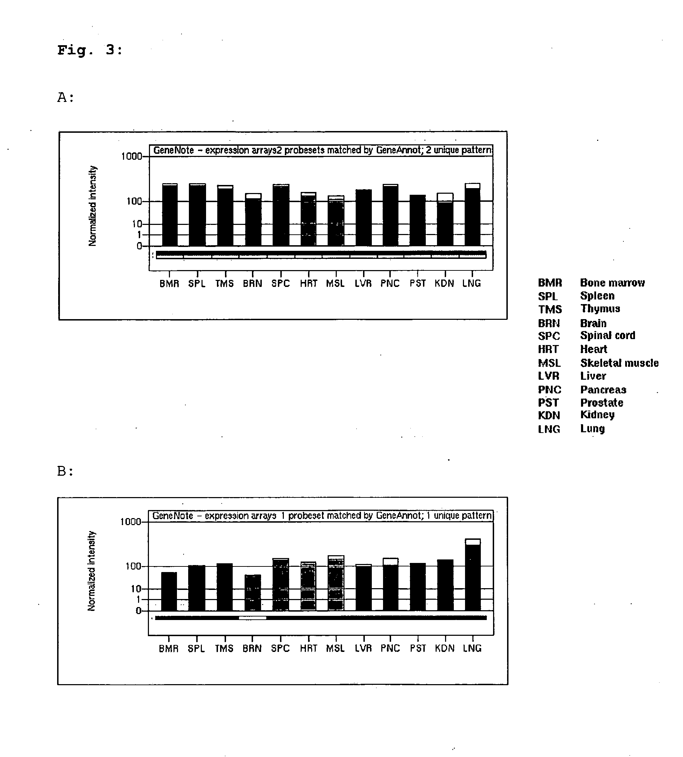

Fig. 3: CDKN2A (A) and CDKN1A (B) gene expression over various tissues: Barplots depict gene expression levels of CDKN2A and CDKN1A in 12 different human tissues as determined by microarray technology.

### Examples:

An estimate of "biological" organ age as derived from markers of cellular senescence in zero hour biopsies allows an accurate prediction of allograft function one year after transplantation. Clinical data were collected.retrospectively from 35 patients with a functioning deceased donor graft and telomere length and mRNA expression levels of the cell cycle inhibitors CDKN2A and CDKN1A were determined in pre-implantation biopsies. In uni- and multivariate regression analysis parameters associated with serum creatinine after one year were identified and used for deriving predictive models. The accuracy of these models was then evaluated in a prospective study including 19 patients.

The predictive power of donor age was similar to a combination of telomere length and CDKN2A (r²=0.34 and 0.33) for the retrospectively collected sample set, both models being inferior to a model holding data on donor age and CDKN2A (r²=0.44, p = 0.0135). In the prospective sample set a significant correlation of predicted and measured creatinine values after one year was achieved only for models including CDKN2A and telomere length or age (r = 0.62, p < 0.001 and r = 0.59, p < 0.001) but not for age alone. The significant superiority of the models including molecular markers was also confirmed in a cross-validation analysis including data from all subjects. Therefore, chronological donor age is a strong predictor of renal allograft function one year after transplantation, but clinically relevant additional information can be obtained from the determination of telomere length and CDKN2A gene expression in zero hour biopsies.

### Example 1: Patients and sample preparation

All patients involved in this study received a deceased donor transplant. The retrospective part of the study included 35 patients, the prospective part 19 subjects. A pre-implantation biopsy core, which was obtained from all subjects as part of the routine renal transplant procedure, was bisected and stored either in 4 % formaldehyde solution for subsequent histological evaluation or in RNAlater^{®} (Ambion, Texas, USA). The formaldehyde fixed paraffin embedded biopsies were processed according to standard procedures and 4 serial 2 µm sections were stained with Hematoxilin and Eosin, SPAS (Perjodic Acid Schiff), Jones's Methenamin Silver and a trichrome stain (acidic fuchsin orange-G). The percentage of sclerotic glomeruli was calculated and the extent of arteriolar hyalinosis, interstitial inflammation, interstitial fibrosis and tubular atrophy was determined using a semi quantitative 4 grade scoring system (0=absent, 1=mild, 2=moderate, 3=severe). The RNA sample stored later was used for determining the telomere length and gene expression.

In addition to the molecular markers the following data were obtained for each patient in the retrospective study (table 1): Serum creatinine and estimated glomerular filtration rate (eGFR) according to the MDRD formula at one year after transplantation, donor/recipient age and gender, percentage of preformed panel reactive antibodies at transplantation, cold ischemia time and number of HLA A-, B- and Dr- mismatches. Postoperative acute allograft failure was defined as the need for dialysis within the first week after transplantation. The incidence of an biopsy proven acute rejection as well as the type of immunosuppressive medication during the follow up time was also recorded.

The parameters used for analysis of the prospective study population are presented in table 2.

### Example 2: Isolation and Quantification of DNA and RNA

Genomic DNA and total RNA were extracted using commercially available kits (DNeasy^{®}Tissue Kit, Quiagen, Hilden, Germany; TRI Reagent^{®}, Molecular Research Center, Cincinnati, USA). Prior to quantitative PCR experiments the concentration of genomic DNA obtained from each sample was adjusted to 2.5 ng/µl by using a fluorescence based kit (PicoGreen^{®} dsDNAQuantitation Kit, Molecular Probes, Eugene, Oregon, USA). Total RNA was determined by absorbance at 260 nm and equated to a concentration of 0.1 µg/µl. Genomic DNA and total RNA extracted from the healthy part of a kidney, which had to be removed from a 33 year old male patient because renal cell cancer was used as a standard.

### Example 3: Determination of Telomere Length by Real-Time PCR

Telomere length values were measured from DNA by a quantitative PCR assay that determines the relative ratio of telomere repeat copy number to single-copy gene copy number (T/S ratio) in experimental samples as compared with a reference DNA sample, as described in detail by Cawthon (Nucleic Acids Res, 2002; 30:e47). In brief, separate duplicate PCR experiments were performed for telomere (T) and 36B4 (a single copy gene, S). The primer pair sequences for telomere PCR were (5' to 3'): tel1b CGGTTTGTTTGGGTTTGGGTTTGGGTTTGGGTTTGGGTT and tel2b GGCTTGCCTTAC-CCTTACCCTTACCCTTACCCTTACCCT. Sequences for 36B4: 36B4 forward CAGCAAGTGGGAAGGTGTAATCC; 36B4 reverse CCCATTCTATCATCAACGGGTACAA. Telomere length was also measured by Southern blot in 7 samples (TeloTAGGG Telomere Length Assay, Roche Diagnostics, Germany). The results correlated highly with the ones obtained by Real Time PCR (r² = 0.89).

### Example 4: Determination of CDKN2A and CDKN1A mRNA Expression by Real Time PCR

Samples were transcribed into cDNA and determination of gene expression levels of CDKN2A and CDKN1A was performed utilizing a commercially available pre-designed primer set and TaqMan Mix (Applied Biosystems, Foster City, CA, USA). Thermal cycling profiles started with 2 minutes at 50°C (RNAse inhibitors activation) and 10 minutes at 95°C to activate the polymerase. Repeated cycles were performed 40 times at 95°C for 15 seconds, followed by 60°C for 1 minute. Samples were run in duplicate for CDKN2A and CDKN1A and calculations were conducted following the ddCt method. Except for 18s RNA (Applied Biosystems, Foster City, CA, USA) all other tested housekeeping genes (GAPDH, β-Actin and β-2-microglobulin) showed signs of co-regulation and were therefore not considered appropriate for conclusive experiments.

All experiments were carried out using the ABI PRISM 7500 and evaluated by the SDS 1·9·1 software package and GraphPad Prism^{®}.

### Example 5: Statistical Analysis The demographic data given in tables 1 and 2 are presented

as mean ± SD or median and IQR. The t-test or Wilcoxon rank sum test was used to evaluate differences between the transplant centres as well as between the retrospective and the prospective dataset. Pairwise scatterplots were constructed for donor age, telomere length, and expression values of CDKN2A and CDKN1A. The Spearman correlation coefficient was used as quantitative measure to describe correlations between investigated variables. Univariate and multivariate linear regression analyses were performed to determine the associations of the independent variables in the retrospective study with serum creatinine values 12 months post-transplant. A backward model selection procedure was applied to identify the best regression model. In addition, the predictive power of a model consisting of telomere length and CDKN2A expression was evaluated. The models were checked for linearity, homoscedasticity, and normal distribution of the residuals. No outliers could be detected using Cook's distance measure. Donor and recipient age were considered continuous and are reported on a per decade base. Models using donor age alone, the model derived from backward selection analysis, as well as a model consisting of telomere length and CDKN2A expression were used to predict graft function one year after transplantation derived on the basis of prospectively collected samples. A cross-validation analysis on the whole data set of 54 samples was used to further validate the findings. The relative predictive power of the models for the retrospective analysis is given as r² - or adjusted r² for models holding more than one parameter. The correlation of predicted and measured creatinine values is reported for prospective model validation, as well as the cross-validation analysis.

### Example 6: Results

The characteristics of the study populations are given in table 1 and 2. No centre effect or significant differences between the parameters of the retrospective and the prospective data set were detected.

**Table 1: Experimental and demographic data of the retrospective study population**

| **retrospective dataset (n=35)** | | |
|---|---|---|
| **Parameter** | **Mean** | **SD** |
| serum creatinine at one year | 1·47 | 0·47 |
| telomere length (T/S ratio) | 2·69 | 0·77 |
| CDKN2A mRNA expression | 10·45 | 7·19 |
| CDKN1A mRNA expression | 4·77 | 3·77 |
| donor age (years) | 46·9 | 16·5 |
| recipient age (years) | 52·8 | 13·4 |
| recipient gender (female/male) | 12 / 23 | |
| donor gender (female/male) | 14 / 21 | |
| HLA mismatch (total count) | 1·91 | 1·72 |
| panel reactive antibodies (%) | 2 (0 / 4) | |
| cold ischemia time (hours) | 14·3 | 4·5 |
| acute rejection (no/yes) | 31 / 4 | |
| acute allograft failure (no/yes) | 28 / 7 | |
| Corticosteroids (n) | 32 | |
| Cyclosporine A (n) | 16 | |
| Tacrolimus (n) | 15 | |
| Azathioprine (n) | 3 | |
| Mycophenolate Mofetil (n) | 24 | |
| Rapamycine (n) | 4 | |
| interstitial inflammation (grade) | 0·13 | 0·42 |
| interstitial fibrosis (grade) | 0·25 | 0·51 |
| arteriolar hyalinosis | 0·59 | 0·61 |
| tubular atrophy | 0·41 | 0·50 |
| sclerosed glomeruli (%) | 1·32 | 0·78 |

**Table 2: Experimental and demographic data of the prospective study population**

| **prospective dataset (n=19)** | | |
|---|---|---|
| **Parameter** | **mean** | **SD** |
| serum creatinine at one year | 1·59 | 0·60 |
| telomere length (T/S ratio) | 2·93 | 1·13 |
| CDKN2A mRNA expression | 7·20 | 6·60 |
| donor age (years) | 45·3 | 15·3 |

For the retrospective study associations between Donor Age, Telomere Length, CDKN2A and CDKN1A Expression are given in Fig. 1. A negative, linear and significant correlation was observed between telomere length and donor age and a weak positive correlation between donor age and CDKN2A expression.

To estimate postoperative allograft function and donor/recipient parameters, a univariate regression analysis was performed using serum creatinine at 12 months as dependent variable. Donor age was the single most predictive parameter explaining 33.9% of the variability of one year allograft function (table 3), followed by CDKN2A expression (24.7%) and telomere length (16.2%). Contrary to expectations, the percentage of sclerotic glomeruli was negatively correlated to serum creatinine after one year. However, the maximum number of sclerosed glomeruli per biopsy was 3.5%. No other association was detected although the negative effect of acute postoperative transplant failure on one year organ function almost reached statistical significance.

After a multivariate backward selection analysis, the resulting model consisting of donor age and CDKN2A explained 43.9% of the variability of serum creatinine after one year (p = 0.0135 vs. age alone), the adjusted R² value for a model including telomere length and CDKN2A expression values was 0.33 (p = 0.3936 when compared to the age model).

**Table 3: Univariate regression analysis of the retrospective dataset using serum creatinine at 12 months as the dependent variable (n=35)**

| | **parameter estimate** | **95% CI** | | **p-value** | **adj R²** |
|---|---|---|---|---|---|
| donor age (10 years) | 0·173 | 0·0909; | 0·254 | < 0·001 | 0·339 |
| sclerosed glomeruli (%) | -0·293 | -0·489; | -0·097 | 0·0048 | 0·225 |
| CDKN2A mRNA expression | 0·034 | 0·014; | 0·054 | 0·0014 | 0·247 |
| telomere length (T/S ratio) | -0·267 | -0·465; | -0·070 | 0·0096 | 0·162 |
| acute allograft failure (no/yes) | -0·340 | -0·101; | 0·483 | 0·0900 | 0·057 |
| donor gender (female/male) | 0·194 | -0·137; | 0·526 | 0·2413 | 0·012 |
| acute rejection (no/yes) | -0·272 | -0·784; | 0-240 | 0·2870 | 0·005 |
| panel reactive antibodies (%) | -0·006 | -0·018; | 0·007 | 0·3597 | <0 |
| Tubular atrophy | 0·144 | -0·190; | 0·478 | 0·3847 | <0 |
| arteriolar hyalinosis | -0·109 | -0·380; | 0·163 | 0·4202 | <0 |
| CDKN1A mRNA expression | -0·015 | -0·060; | 0·0290 | 0·4868 | <0 |
| recipient age (10 years) | 0·036 | -0·009; | 0·016 | 0·5589 | <0 |
| recipient gender (female/male) | -0·092 | -0·440; | 0·256 | 0·5940 | <0 |
| interstitial inflammation (grade) | -0·096 | -0·495; | 0·303 | 0·6271 | <0 |
| HLA mismatch (total count) | 0·012 | -0·086; | 0·110 | 0·8040 | <0 |
| interstitial fibrosis (grade) | 0·019 | -0·313; | 0·351 | 0·9060 | <0 |
| cold ischemia time (hours) | -0·001 | -0·039; | 0·036 | 0·9541 | <0 |

A significant and almost identically accurate correlation between predicted and measured creatinine values in the prospective data set could be achieved using the models holding telomere length and CDKN2A expression (pearson r = 0.62, p < 0.001) and donor age and CDKN2A expression (pearson r = 0.59, p < 0.001). No significant correlation could be achieved for donor age alone (pearson r = 0.20, p = 0.407).

Formula using CDKN2A and donor age to predict creatinine values after 12 months: creatinine (12 months post TX) = 0.573235 + (0.013785 * donor age) + (0.023584 * CDKN2A)

Formula using CDKN2A and the telomere length to predict creatinine values after-12 months: creatinine (12 months post TX)= 1.51484 + (-0.12971 * telomere length) + (0.03951 * CDKN2A)

The statistical superiority of these models as compared to using donor age alone was also confirmed in a cross-validation analysis. 35 out of the total of 54 samples were randomly picked, the models consisting of donor age, donor age and CDKN2A, as well as donor age and relative telomere length were generated and finally validated on the remaining 19 samples. This procedure was performed 500 times (figure 2). The mean correlation between predicted and measured creatinine values was 0.60 for the marker model, 0.57 for the model including age and CDKN2A and 0.46 for donor age alone.

Almost identical results were obtained in all analyses when eGFR instead of serum creatinine values were used.

In a univariate analysis chronological donor age, telomere length and CDKN2A expression were significantly associated with serum creatinine 12 months after transplantation, with age alone already explaining about 34% of the observed variability. In a multivariate approach a combination of the two molecular markers was equally predictive as chronological donor age, and a combination of CDKN2A expression and donor age provided even more information. For confirmation, a prospective study was initiated and in this setting only the models holding the molecular senescence markers or donor age and CDKN2A gene expression were significantly associated with serum creatinine, whereas the predictive power of chronological donor age was much lower.. This result was confirmed using data from the whole study population in a cross-validation analysis. Chronic allograft failure per se has been associated with cellular senescence including telomere shortening and CDKN2A induction. If, indeed, chronic allograft nephropathy is a consequence of accelerated aging, the biological organ age at the time of transplantation might be crucial.

## Claims

1. A method of a diagnostic or prognostic assay for determining organ quality, in particular for suitability of organ transplantation, in a donor organ sample, which comprises determining an amount of CDKN2A and the chronological age or telomere length in the sample.

2. Method according to claim 1, **characterized in that** the organ is a kidney.

3. Method according to claim 1, **characterized in that** the organ is selected from bone marrow, heart, bowel, spleen, thymus, spinal cord, muscle, in particular skeletal muscle, liver, pancreas, prostate or lung.

4. Method according to any one of claims 1 to 3, **characterized in that** the sample is a biopsy sample.

5. Method according to any one of claims 1 to 4, comprising the step of providing the donor organ sample.

6. Method of any one of claims 1 to 5 for predicting transplant organ function, in particular as determined by creatinine levels in blood, of a subject which received an organ transplant, comprising determining an amount of CDKN2A and the chronological age or telomere length in the donor organ sample prior to transplantation, correlating a known organ function with CDKN2A and the chronological age or telomere length, and calculating the predicted organ function from the correlation with the determined amounts of CDKN2A and the chronological age or telomere length.

7. Method according to any one of claims 1 to 6, **characterized in that** determining the amount of CDKN2A comprises determining the CDKN2A expression, preferably by determining the CDKN2A mRNA concentration.

8. Method according to any one of claims 1 to 7, **characterized in that** determining the amount of CDKN2A comprises determining the CDKN2A protein concentration.

9. Method of claims 1 to 8 for prediction of organ transplant failure, comprising determining an amount of CDKN2A and the chronological age or telomere length in the donor organ sample, and comparing the determined amounts to amounts of CDKN2A and the chronological age and/or telomere length with organ function after transplantation of healthy patients, wherein the chance of organ transplant failure is increased if the determined amounts in the donor sample are greater than the amounts of the healthy transplant patients.

10. Method according to claim 9, **characterized in that** the organ transplant failure is a chronic organ transplant failure.

11. Set comprising means for the determination of CDKN2A mRNA or CDKN2A protein, preferably CDKN2A mRNA specific PCR primers, CDKN2A hybridizing oligonucleotides or CDKN2A specific antibodies, and means for the determination of telomere length, preferably primers for the determination of the T/S-value.
